# FASCICULE DE BREVET EUROPEEN

(11) **EP 1 352 615 B1**
(45) Date de publication et mention de la délivrance du brevet: **02.11.2005**
(21) Numéro de dépôt: 03290870.9
(22) Date de dépôt: 08.04.2003
(51) Int. Cl.: A61B 18/20

(54) **Dispositif de traitement par émission de flashs lumineux**
Blitzlampenbehandlungsgerät
Flashlight treatment device

(30) Priorité: 08.04.2002 FR 0204313
(43) Date de publication de la demande: 15.10.2003
(60) Demande divisionnaire: 05019414.1
(73) Titulaire: Eurofeedback, 91017 Evry Cedex (FR)
(72) Inventeur: Safraoui, Georges, 91140 Villejust (FR)
(74) Mandataire: Tanty, François

(56) Documents cités:
- WO-A-01/87408
- DE-A- 3 800 291
- DE-C- 875 679
- US-A- 4 745 613
- US-A- 5 287 380
- US-A- 5 720 772
- US-A- 5 735 844
- US-B1- 6 273 883

## Description

La présente invention concerne les dispositifs de traitement par émission de flashs lumineux, notamment pour l'épilation ou des applications thérapeutiques.

La demande de brevet européen EP 0 885 629 décrit un dispositif de traitement comportant une lampe flash et un générateur auquel est reliée la lampe flash. Ce générateur comporte un condensateur chargé sous une tension comprise entre 100 et 1000 volts et dont la capacité est comprise entre 10 et 100 mF ainsi qu'un interrupteur électronique constitué par un seul transistor IGBT.

Un tel dispositif n'offre pas entière satisfaction, car en cas de défaillance de l'interrupteur électronique, toute l'énergie électrique stockée dans le condensateur est susceptible d'être transformée en lumière, ce qui peut occasionner une brûlure de la zone traitée.

En effet, l'énergie stockée représente par exemple entre deux et quatre fois celle qui est nécessaire à l'émission d'un flash, de sorte que la fluence accidentelle en cas de défaillance de l'interrupteur électronique peut atteindre 100 J/cm² environ alors qu'au-delà de 30 J/cm² des lésions peuvent apparaître.

Le brevet US 5 720 772 décrit un dispositif de traitement comportant un générateur comprenant trois condensateurs de capacités respectives 1 mF, 10 mF et 100 mF, associés chacun à un interrupteur et à une self. Les interrupteurs peuvent être commandés séparément ou séquentiellement. Une densité d'énergie de 30 à 100 J/cm² peut être obtenue sur la peau. En cas de défaillance de l'interrupteur associé au condensateur de 100 mF, une densité d'énergie excessive est susceptible d'être délivrée.

Il existe par conséquent un besoin pour améliorer encore la sécurité des dispositifs de traitement par émission de flashs lumineux.

Selon un aspect de l'invention, le générateur électrique comporte plusieurs interrupteurs électroniques associés chacun à un condensateur respectif, chaque interrupteur électronique pouvant être commandé d'un état non passant à un état passant permettant à de l'énergie électrique accumulée dans le condensateur associé d'être déchargée dans la lampe, la capacité de chaque condensateur étant suffisamment faible pour qu'en cas de défaillance de l'interrupteur électronique correspondant et la décharge dans la lampe de la totalité de l'énergie électrique accumulée dans le condensateur, le flash lumineux ainsi généré ait une densité d'énergie suffisamment faible pour ne pas occasionner de risque de lésion indésirable de la zone traitée.

Par « condensateur », il faut comprendre au sens de la présente invention un composant capacitif unique ou un ensemble de composants capacitifs reliés électriquement en parallèle et/ou en série de manière à être équivalent à un composant capacitif unique ayant la capacité voulue.

De même, par « interrupteur électronique », il faut comprendre au sens de la présente invention un ou plusieurs composants de commutation assemblés en série et/ou parallèle. Ainsi, un interrupteur électronique selon l'invention peut être constitué par un seul transistor, par exemple du type IGBT, ou par plusieurs transistors assemblés en parallèle et/ou en série de façon à présenter le pouvoir de commutation souhaité.

La capacité de chaque condensateur est suffisamment faible pour que la fluence du flash lumineux provoqué par la décharge dans la lampe de la totalité de l'énergie accumulée dans ce condensateur soit inférieure ou égale à environ 30 J/cm².

Le générateur peut comporter entre trois et six condensateurs associés à des interrupteurs électroniques respectifs, et chaque condensateur présente une capacité inférieure ou égale à 15 mF, par exemple voisine de 10 mF.

Le dispositif de traitement comporte avantageusement un dispositif de contrôle, notamment pour commander les interrupteurs électroniques.

Ces derniers peuvent être commandés simultanément ou non en commutation de l'état non passant à l'état passant. Ils peuvent être commandés simultanément ou non de l'état passant à l'état non passant lorsque la quantité d'énergie désirée a été déchargée dans la lampe.

Lorsque les interrupteurs électroniques sont commutés simultanément de l'état non passant à l'état passant, tous les condensateurs associés peuvent décharger de l'électricité simultanément dans la lampe.

Lorsque les interrupteurs électroniques sont commutés non simultanément de l'état non passant à l'état passant, ils peuvent être commutés séquentiellement, avec recoupement des intervalles de commutation ou non. Par exemple, les interrupteurs peuvent être commutés séquentiellement de l'état non passant à l'état passant puis à l'état non passant de manière à décharger successivement l'énergie électrique contenue dans chacun des condensateurs, l'énergie électrique d'un seul condensateur à la fois étant déchargée dans la lampe. Cela peut se traduire par une succession de flashs lumineux plus ou moins rapprochés dans le temps.

Le dispositif de contrôle peut être configuré pour commuter chaque interrupteur électronique lorsqu'une énergie prédéterminée a été déchargée dans la lampe. En particulier, le dispositif de contrôle peut être programmable de manière à permettre à un utilisateur de programmer l'énergie du flash lumineux, cette programmation pouvant s'effectuer par exemple en appuyant sur des touches correspondant chacune à une nature de traitement, ce qui évite d'avoir à entrer des valeurs numériques.

Le dispositif de contrôle peut recevoir une information relative au fonctionnement de chaque interrupteur électronique, notamment une information représentative du courant circulant au travers de chaque interrupteur électronique. Cette information peut servir à commander l'interruption de la décharge du condensateur associé lorsque l'énergie désirée a été déchargée dans la lampe. Elle peut servir également à permettre au dispositif de contrôle de détecter une anomalie de fonctionnement.

Selon un autre aspect de l'invention, le dispositif de traitement comporte un dispositif de détection de défaut d'isolation, ce qui offre une sécurité supplémentaire.

Ce dispositif de détection de défaut d'isolation peut être configuré pour détecter une variation de potentiel aux bornes de deux résistances reliées en série, ayant un point milieu relié à la terre et des bornes reliées aux deux polarités des condensateurs. Un tel dispositif de détection de défaut d'isolation peut être configuré pour détecter un courant de fuite inférieur à 100 µA, voire inférieur à 50 µA.

En cas de détection d'un courant de fuite, le fonctionnement du dispositif de traitement est empêché.

Lorsqu'un liquide de refroidissement isolant électrique, par exemple de l'eau déminéralisée, est utilisé pour refroidir la lampe, ce liquide venant en contact électrique entre la terre et l'une des deux polarités des condensateurs, le dispositif de détection de défaut d'isolation peut permettre de détecter une contamination du liquide. Cela peut permettre également de détecter l'utilisation d'un liquide non adapté, non isolant électrique. Par exemple, si au lieu de remplir le circuit de refroidissement avec de l'eau déminéralisée, de l'eau courante est utilisée, le dispositif de détection de défaut d'isolation détectera un courant de fuite à travers le liquide et le fonctionnement du dispositif de traitement sera empêché.

Le dispositif de traitement peut être dépourvu d'alimentation électrique en mode simmer de la lampe flash. Cela peut permettre d'éviter l'alimentation permanente de la pièce à main avec une tension relativement élevée et de réduire le rayonnement électromagnétique de l'appareil ainsi que les risques liés à la présence de haute tension.

Selon un aspect de l'invention, au moins une bobine saturable peut être reliée électriquement en série avec la lampe. Cette bobine saturable est configurée pour permettre l'établissement au début de la décharge des condensateurs, dans un premier temps, d'un courant de « pseudo-simmer » dans la lampe. Un tel courant permet de prolonger la durée de vie de la lampe flash, car le plasma peut s'établir bien au centre du tube avant son expansion.

La bobine permet également de réduire les pertes de commutation dans les interrupteurs électroniques.

Selon un autre aspect de l'invention, le dispositif de contrôle est configuré pour recevoir une information d'au moins un capteur apte à délivrer une information représentative d'un contact ou d'une proximité de la pièce à main avec la zone à traiter et de préférence représentative également de la pression avec laquelle une pièce à main contenant la lampe est appliquée sur la zone à traiter.

La pièce à main peut comporter une fenêtre de sortie de la lumière vers la zone à traiter et le capteur peut être disposé de manière à détecter la pression exercée par cette fenêtre contre la peau.

Le dispositif de contrôle peut être configuré pour ne pas permettre l'émission d'un flash tant que la pression exercée par la pièce à main sur la zone à traiter est inférieure ou égale à une limite prédéterminée. Cela permet d'éviter l'émission accidentelle d'un flash lumineux alors que la pièce à main n'est pas appliquée contre la zone à traiter.

Par ailleurs, le dispositif de traitement peut également être configuré pour ne pas permettre l'émission d'un flash lumineux tant que la pression d'application de la pièce à main sur la zone à traiter n'est pas comprise entre des limites inférieure et supérieure (qui peut être infinie) prédéterminées, pour une nature de traitement donnée.

Cela peut permettre, lors d'une utilisation de l'appareil en épilation, de n'autoriser l'émission d'un flash lumineux que lorsque la pression de la pièce à main sur la peau est suffisante pour chasser l'hémoglobine.

Par contre, lors d'un traitement vasculaire, l'énergie déchargée dans la lampe peut ne pas être la même que dans le cas de l'épilation, le flash lumineux étant par exemple différent en spectre et en énergie et la pression exercée par la pièce à main sur la zone traitée doit être plus faible pour ne pas chasser l'hémoglobine ; le dispositif de contrôle pourra n'autoriser l'émission d'un flash lumineux que lorsque la pression de la pièce à main sur la peau est comprise dans un intervalle prédéterminé.

Selon un autre aspect de l'invention, le dispositif de traitement comporte au moins un détecteur apte à détecter une ouverture d'un capot amovible du générateur permettant d'accéder aux circuits électroniques, et le dispositif de traitement est configuré pour mémoriser l'ouverture du capot et empêcher qu'en l'absence d'une intervention prédéterminée, effectuée par une personne autorisée, le dispositif de traitement puisse fonctionner à nouveau. On évite ainsi une intervention non autorisée sur les circuits électroniques du dispositif de traitement, susceptible notamment d'affecter la sécurité.

Le dispositif de traitement peut comporter un lecteur d'un support d'unités de crédit, par exemple un lecteur de carte à puce, et le dispositif de traitement peut être configuré pour décrémenter un compte utilisateur à chaque utilisation, par exemple à chaque émission de flash. Cela peut permettre par exemple de facturer un centre médical ou d'esthétique en fonction de l'utilisation effective du dispositif de traitement.

L'invention a encore pour objet le générateur défini plus haut, considéré isolément.

L'invention a encore pour objet, selon un autre de ses aspects, un dispositif de traitement par émission de flashs lumineux, notamment pour l'épilation ou des applications thérapeutiques, comportant au moins une lampe flash et un générateur auquel est reliée la lampe flash, ce dispositif étant caractérisé par le fait qu'il comporte un dispositif de contrôle configuré pour recevoir au moins une information d'un capteur apte à délivrer une information représentative d'un contact ou de la proximité d'une pièce à main contenant la lampe avec la zone à traiter et de préférence représentative également de la pression avec laquelle la pièce à main est appliquée contre la zone à traiter.

Un tel dispositif permet d'éviter le départ de flashs lumineux accidentellement ailleurs que sur la zone à traiter et permet également de n'autoriser l'émission de flashs lumineux que dans certaines conditions de pression de la pièce à main sur la zone traitée, ce qui est utile lorsque des traitements de différentes natures, par exemple d'épilation ou vasculaire, peuvent être programmés.

L'invention a encore pour objet, selon un autre de ses aspects, un dispositif de traitement par émission de flashs lumineux, notamment pour l'épilation, comportant au moins une lampe flash et un générateur auquel est reliée la lampe flash, ce dispositif étant caractérisé par le fait que la lampe est reliée au générateur par l'intermédiaire d'au moins une bobine saturable.

Un tel dispositif permet d'augmenter la durée de vie de la lampe, comme expliqué plus haut et d'éviter l'utilisation d'une alimentation en mode simmer de la lampe.

L'invention pourra être mieux comprise à la lecture de la description détaillée qui va suivre, d'un exemple de mise en oeuvre non limitatif, et à l'examen du dessin annexé, sur lequel :
- la figure 1 représente de manière schématique, en perspective, un dispositif de traitement,
- la figure 2 représente de manière schématique et simplifiée un étage de sortie pouvant être utilisé,
- la figure 3 illustre l'utilisation d'un disjoncteur différentiel,
- la figure 4 illustre l'utilisation d'une bobine saturable, reliée électriquement en série avec la lampe flash,
- la figure 5 représente de manière schématique l'évolution du courant IL de la lampe et de la tension V_{G} de l'étage de sortie en fonction du temps,
- la figure 6 représente isolément et schématiquement un capteur de pression,
- la figure 7 illustre la possibilité de conditionner l'émission d'un flash lumineux à des conditions de pression particulières de la pièce à main sur la zone traitée, et
- la figure 8 représente un exemple de détecteur d'ouverture.

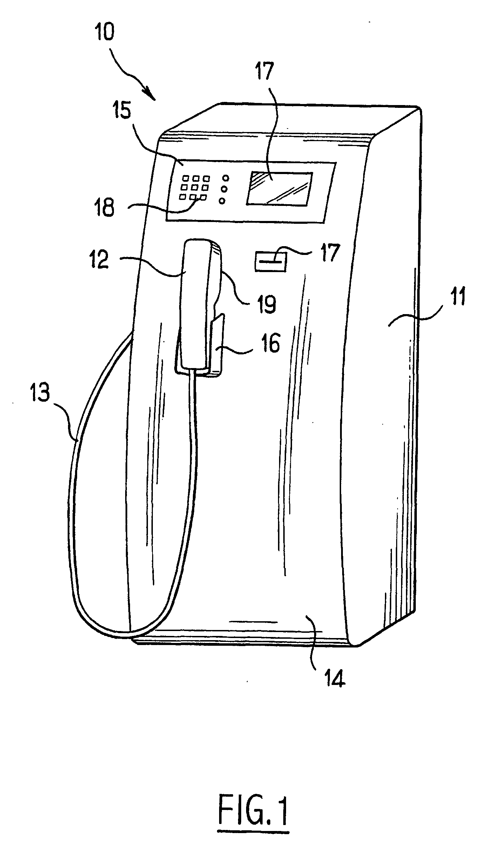

On a représenté sur la figure 1 un dispositif de traitement 10 comportant un générateur 11 et une pièce à main 12 reliée au générateur 11 par un flexible 13.

Le générateur 11 peut comporter, comme dans l'exemple illustré, une face avant 14 comportant un panneau de contrôle 15.

La face avant 14 peut être pourvue d'un support 16 permettant d'accrocher la pièce à main 12 en l'absence d'utilisation et d'une fente 17 permettant l'introduction d'une carte à puce.

Le panneau de contrôle 15 peut comporter un dispositif d'affichage 17 ainsi qu'un clavier 18 permettant d'entrer des informations concernant par exemple la nature du traitement.

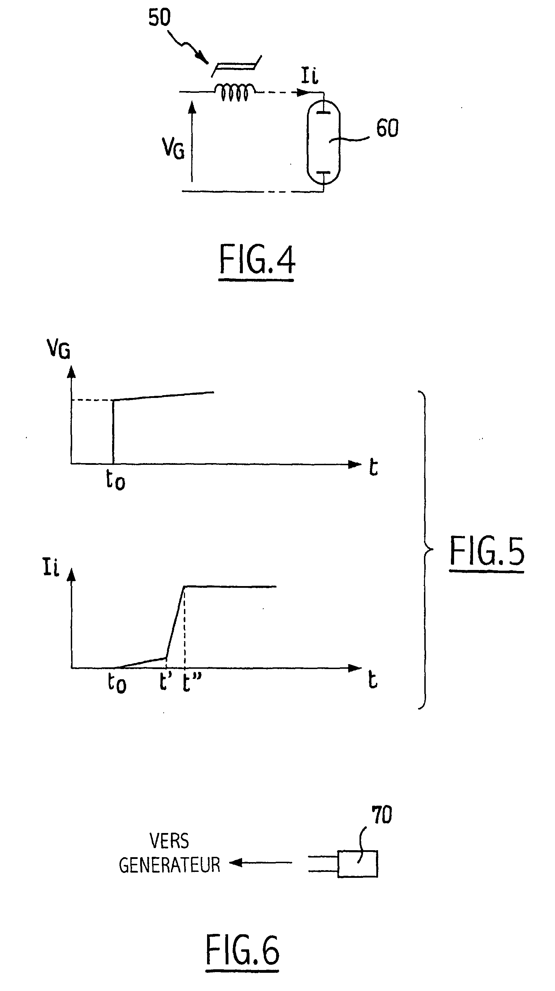

La pièce à main 12 comporte une lampe flash 60 représentée schématiquement à la figure 4, apte à émettre une lumière incohérente vers la zone à traiter.

La lampe flash est par exemple constituée par un tube xénon de 5 ou 6 mm de diamètre.

La lampe flash peut être refroidie, comme c'est le cas dans l'exemple illustré, par un liquide de refroidissement qui est mis en circulation par une pompe disposée à l'intérieur du générateur 11 et circulant par des conduits du flexible 13. La pièce à main comporte une fenêtre de sortie 19 par laquelle la lumière provenant de la lampe est émise vers la peau.

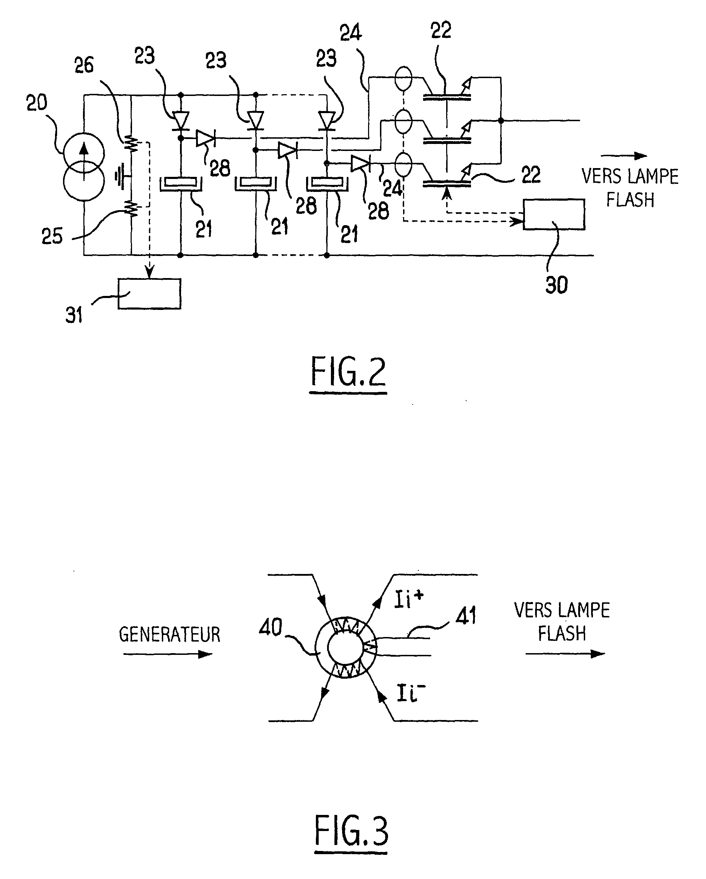

Le générateur 11 comporte une alimentation électrique 20 à moyenne tension (quelques centaines de volts), représentée schématiquement sur la figure 2 et qui est par exemple réalisée conformément aux enseignements de la demande de brevet européen EP 0 772 286 au nom de la demanderesse.

On voit sur la figure 2 que le générateur 11 comporte un étage de sortie comportant une pluralité de condensateurs 21 associés respectivement chacun à un interrupteur électronique 22.

Chaque condensateur 21 peut être constitué par exemple, comme dans l'exemple illustré, par un composant capacitif électrochimique unique ayant 10 mF de capacité.

Chaque interrupteur électronique 22 peut être constitué par exemple par un transistor de puissance IGBT.

Les bornes négatives des condensateurs 21 sont reliées électriquement à la borne négative de l'alimentation 20 tandis que les bornes positives sont reliées, par l'intermédiaire de diodes 23 respectives à la borne positive de l'alimentation 20.

Le générateur 11 comporte un dispositif de contrôle 30 configuré notamment pour commander les interrupteurs électroniques 22.

Ce dispositif de contrôle 30 reçoit une information représentative du courant qui parcourt les conducteurs 24 qui relient les cathodes des diodes 23 aux interrupteurs électroniques 22 correspondants. Des diodes 28 peuvent être disposées en série avec les conducteurs 24 pour autoriser une commutation séquentielle des interrupteurs 22.

La mesure du courant dans chacun des conducteurs 24 peut s'effectuer par exemple en mesurant la tension aux bornes d'une résistance (non représentée) parcourue par le courant de décharge du condensateur ou par tout autre moyen, par exemple un capteur inductif.

Le générateur 11 comporte également un dispositif de détection de défaut d'isolation 31 apte à détecter une variation de la tension aux bornes de chacune de deux résistances 25 et 26 reliées électriquement en série entre les bornes de l'alimentation 20 et dont le point milieu 27 est relié à la terre.

L'étage de sortie est flottant et en l'absence de courant de fuite les tensions aux bornes des résistances 25 et 26, qui présentent une valeur très élevée, par exemple une valeur identique de l'ordre de quelques MΩ, sont sensiblement égales.

En cas de déséquilibre lié à l'existence d'un courant de fuite, par exemple en cas de contact de la polarité négative de l'alimentation 20 par un utilisateur, un déséquilibre apparaît au niveau des tensions mesurées aux bornes des résistances 25 et 26, et peut être détecté par le dispositif de détection de défaut d'isolation 31.

Des courants de fuite inférieurs à 50 µA peuvent être ainsi détectés.

Sur la figure 2 n'ont été représentés que trois couples de condensateurs 21 et d'interrupteurs électroniques 22 mais bien entendu on ne sort pas du cadre de la présente invention lorsque plus ou moins de trois condensateurs 21 et interrupteurs électroniques correspondants 22 sont utilisés.

Le dispositif de détection de défaut d'isolation 31 permet également, de manière avantageuse, de détecter une contamination du liquide servant à refroidir la lampe dans la pièce à main 12 ou l'emploi d'un liquide autre que le liquide préconisé, à savoir de l'eau déminéralisée en l'espèce.

Outre le dispositif de détection de défaut d'isolation 31, un disjoncteur différentiel 40, représenté schématiquement à la figure 3, peut être utilisé. Ce disjoncteur différentiel 40 peut comporter un anneau magnétique sur lequel sont bobinés les conducteurs de polarité + et - provenant de l'étage de sortie de la figure 2.

Un enroulement 41 sur l'anneau permet de détecter un déséquilibre des courants I_{L}⁺ et I_{L}⁻ correspondants à chacune des polarités.

Le générateur 11 est configuré de manière à empêcher le fonctionnement en cas de déséquilibre.

Selon un aspect de l'invention, une bobine saturable 50 est reliée électriquement en série avec la lampe flash 60, comme illustré sur la figure 4.

Une telle bobine saturable peut comporter une ferrite magnétisable. Au début de l'impulsion de courant provenant de l'étage de sortie, c'est-à-dire à l'instant t₀ sur la figure 5, la tension V_{G} aux bornes de l'étage de sortie peut passer de 0 à environ 300 volts. Le courant IL évolue, entre les instants t₀ et t' relativement lentement, tant que la ferrite n'est pas saturée. Une fois cette dernière saturée, le courant évolue plus rapidement, entre les instants t' et t" pour atteindre sa valeur maximum. La bobine saturable permet, la ferrite étant saturée, d'obtenir un temps de montée maximum du courant d'environ 1A/µs entre les instants t' et t", ce qui permet de réduire les pertes de commutation dans les interrupteurs électroniques 22.

Le courant magnétisant qui parcourt la lampe 60 entre les instants t et t' permet de créer un « pseudo-simmer » dans le tube, lequel prolonge sa durée de vie en permettant au plasma de bien s'établir au centre du tube avant son expansion.

Le rapport entre les pentes des montées de courant entre les instants t₀ et t' d'une part et t' et t" d'autre part peut être de l'ordre de vingt.

Le générateur 11 peut avantageusement être configuré pour recevoir une information d'au moins un capteur 70 représenté isolément sur la figure 6, apte à délivrer une information (par exemple numérique ou analogique, notamment une valeur de résistance, de tension, de courant, un mot binaire) représentative de la pression exercée par la pièce à main 12 sur la zone traitée.

Le dispositif de contrôle 30 peut être configuré de manière à empêcher l'émission d'un flash lumineux tant qu'une pression prédéterminée n'est pas exercée par la fenêtre de sortie 19 sur la peau.

Cela permet d'éviter le déclenchement accidentel d'un flash lumineux ailleurs qu'en direction de la peau.

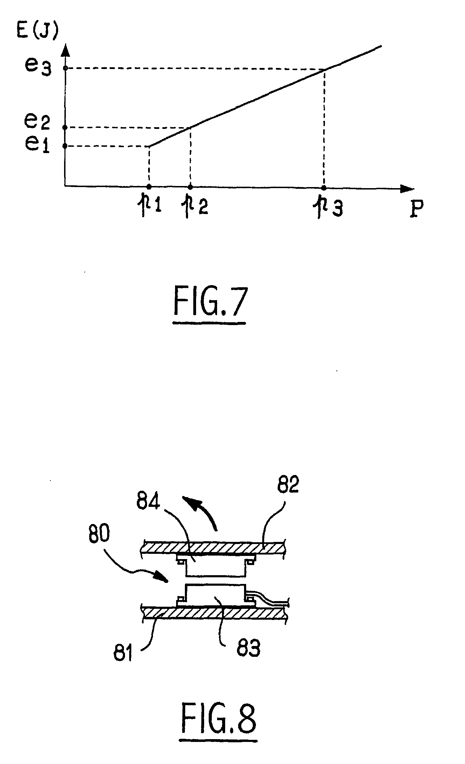

Cela peut également permettre, comme illustré à la figure 7, de n'autoriser l'émission d'un flash lumineux, pour une énergie pré-programmée, que lorsque la pression exercée par la pièce à main sur la zone à traiter est comprise entre des valeurs prédéterminées.

Ainsi, par exemple, le domaine d'énergie E compris entre e₁ et e₂ peut correspondre à l'énergie recommandée pour effectuer un traitement vasculaire et les énergies supérieures à e₃ peuvent correspondre aux énergies requises pour l'épilation.

Lorsque la pression P exercée par la pièce à main 12 sur la peau est comprise entre les valeurs p₁ et p₂ et que l'énergie à délivrer est comprise entre e₁ et e₂, le dispositif de contrôle 30 peut autoriser l'émission d'un flash. Par contre, lorsque la pression P est inférieure à p₁ ou supérieure à p₂, le dispositif de contrôle 30 empêche l'émission d'un flash.

Lorsque le dispositif de traitement 10 est programmé pour l'épilation et que l'énergie lumineuse à délivrer est supérieure à e₃, le dispositif de contrôle 30 peut empêcher l'émission d'un flash tant que la pression exercée P est inférieure à la valeur p₃.

Le dispositif de contrôle 30 peut également être configuré, le cas échéant, pour ajuster l'énergie E du flash lumineux en fonction de la pression P exercée au moment où le tir est déclenché, en calculant en fonction de la pression P l'énergie E correspondante la plus appropriée, par exemple en respectant une loi prédéterminée qui est par exemple une droite comme illustré.

La prise en compte de la pression d'application de la fenêtre de sortie sur la peau par le dispositif de traitement 10 permet d'amener l'opérateur à devoir exercer sur la peau du patient, une pression suffisamment importante pour chasser l'hémoglobine de la zone traitée lors de l'épilation et, dans le cas d'un traitement vasculaire, d'empêcher l'opérateur d'exercer une pression trop importante qui aurait pour conséquence de chasser l'hémoglobine de la zone à traiter et de réduire ainsi l'efficacité du flash.

Le dispositif de traitement 10 comporte avantageusement un détecteur 80, représenté schématiquement à la figure 8, fixé sur une paroi intérieure 81 du générateur 11 et sensible à l'ouverture d'un capot 82 permettant d'accéder à ses circuits électroniques.

Dans l'exemple illustré, ce détecteur 80 comporte d'une part une partie fixe 83 constituée par exemple par un contact de type ILS et d'autre part un aimant 84 fixé sur le capot 82 de manière à permettre à la partie fixe 83 de détecter l'enlèvement du capot 82.

La partie fixe 83 est reliée au dispositif de contrôle 30 et ce dernier est configuré de manière à mémoriser l'ouverture du capot 82 et à empêcher, en l'absence d'une intervention prédéterminée effectuée par une personne autorisée, le dispositif de traitement de fonctionner à nouveau.

Le dispositif de contrôle 30 peut être configuré pour décrémenter une carte à puce introduite dans le lecteur 17, afin par exemple de diminuer un compte d'unités de crédit mémorisées dans la puce. Le générateur 11 est alors configuré de manière à ce que lorsque le compte ne comporte plus d'unités de crédit, l'émission d'un flash lumineux est empêchée.

Le dispositif de traitement 10 peut s'utiliser de la manière suivante.

L'opérateur introduit une carte à puce chargée dans le lecteur 17 et programme à l'aide du clavier 18 la nature du traitement souhaité, par exemple un traitement retardant la repousse des poils, d'épilation définitive, un traitement anti-rides ou vasculaire. Eventuellement, l'opérateur peut entrer directement la valeur de densité d'énergie souhaitée, mais le dispositif de contrôle 30 peut être configuré pour commander les interrupteurs électroniques 22 de façon à cesser la décharge des condensateurs 21 lorsque l'énergie délivrée par la lampe 60 atteint une valeur prédéterminée, qui peut être fixée par exemple à 20 J/cm² par sécurité.

La pièce à main 12 comporte une gâchette (non représentée) qui permet à l'opérateur de déclencher l'émission d'au moins un flash lumineux.

Comme expliqué précédemment, le générateur 11 empêche l'émission d'un flash même lorsque la gâchette est actionnée, en l'absence d'une pression suffisante exercée par la pièce à main 12 sur la peau ou lorsque la pression exercée ne correspond pas à la pression requise, compte tenu de la nature du traitement.

L'invention n'est pas limitée à l'exemple qui vient d'être décrit et on ne sort pas du cadre de la présente invention lorsque le générateur ne comporte qu'une partie seulement des caractéristiques qui ont été décrites.

Le capteur de pression peut ainsi être supprimé ou remplacé par tout autre type de capteur, notamment un capteur de contact ou de distance, ce qui peut réduire le risque d'émission d'un éclair dans une direction autre que la zone à traiter.

Dans toute la description y compris les revendications, l'expression « comportant un » doit être comprise comme étant synonyme de « comportant au moins un », sauf si le contraire est spécifié.

## Revendications

1. Dispositif de traitement (10) par émission de flashs lumineux, comportant une lampe flash (60) et un générateur (11) auquel la lampe est reliée, le générateur (11) comportant plusieurs interrupteurs électroniques (22) associés chacun à un condensateur (21) respectif, chaque interrupteur électronique (22) pouvant être commandé d'un état non passant à un état passant permettant à de l'énergie électrique accumulée dans le condensateur associé d'être déchargée dans la lampe, **caractérisé par le fait que** la capacité de chaque condensateur (21) est inférieure ou égale à 15 mF pour qu'en cas de défaillance de l'interrupteur électronique correspondant et la décharge dans la lampe (60) de la totalité de l'énergie électrique accumulée dans le condensateur (21), le flash lumineux ainsi généré ait une densité d'énergie inférieure ou égale à 30 J/cm² pour ne pas occasionner de risque de lésion indésirable de la zone traitée.

2. Dispositif de traitement selon la revendication précédente, **caractérisé par le fait qu'**il comporte un dispositif de contrôle (30) pour commander les interrupteurs électroniques (22).

3. Dispositif de traitement selon la revendication 2, **caractérisé par le fait que** les interrupteurs électroniques (22) sont commandés simultanément en commutation de l'état non passant à l'état passant.

4. Dispositif de traitement selon l'une des revendications 2 et 3, **caractérisé par le fait que** le dispositif de contrôle (30) est configuré pour commuter de l'état passant à l'état non passant les interrupteurs électroniques lorsqu'une énergie prédéterminée a été déchargée dans la lampe.

5. Dispositif de traitement selon l'une quelconque des revendications 2 à 4, **caractérisé par le fait que** le dispositif de contrôle est programmable de manière à permettre à un utilisateur de programmer l'énergie du flash lumineux, cette programmation pouvant s'effectuer en appuyant sur des touches (18) correspondant chacune à une nature de traitement.

6. Dispositif de traitement selon l'une quelconque des revendications précédentes, **caractérisé par le fait qu'**il comporte un dispositif de détection de défaut d'isolation (31).

7. Dispositif de traitement selon la revendication précédente, **caractérisé par le fait que** le dispositif de détection de défaut d'isolation (31) est configuré pour détecter une variation de potentiel aux bornes de deux résistances (25, 26) reliées en série, ayant un point milieu relié à la terre et des bornes reliées aux deux polarités d'une alimentation (20) des condensateurs.

8. Dispositif de traitement selon l'une des revendications 6 et 7, **caractérisé par le fait que** le dispositif de détection de défaut d'isolation (31) est configuré pour détecter un courant de fuite inférieur à 100 µA, de préférence inférieur à 50 µA.

9. Dispositif de traitement selon l'une quelconque des revendications précédentes, **caractérisé par le fait qu'**un liquide de refroidissement isolant électrique, notamment de l'eau déminéralisée, est utilisé pour refroidir la lampe, ce liquide venant en contact électrique entre la terre et l'une des deux polarités des condensateurs.

10. Dispositif de traitement selon l'une quelconque des revendications précédentes, **caractérisé par le fait qu'**il est dépourvu d'alimentation électrique en mode simmer de la lampe flash (60).

11. Dispositif de traitement selon l'une quelconque des revendications précédentes, **caractérisé par le fait qu'**au moins une bobine saturable (50) est reliée électriquement en série avec la lampe (60).

12. Dispositif de traitement selon l'une quelconque des revendications précédentes, **caractérisé par le fait que** le dispositif de contrôle est configuré pour recevoir une information d'au moins un capteur (70) apte à délivrer une information représentative d'un contact ou d'une proximité de la pièce à main avec la zone à traiter et de préférence représentative également de la pression avec laquelle une pièce à main (12) contenant la lampe est appliquée sur la zone à traiter.

13. Dispositif de traitement selon l'une quelconque des revendications précédentes, **caractérisé par le fait qu'**il est configuré pour ne pas permettre l'émission d'un flash tant que la pression exercée par la pièce à main (12) sur la zone à traiter est inférieure ou égale à une limite prédéterminée.

14. Dispositif de traitement selon l'une quelconque des revendications précédentes, **caractérisé par le fait qu'**il est configuré pour ne pas permettre l'émission d'un flash lumineux tant que la pression d'application de la pièce à main (12) sur la zone à traiter n'est pas comprise entre des limites inférieure et supérieure prédéterminées, pour une nature de traitement donnée.

15. Dispositif de traitement selon l'une quelconque des revendications précédentes, **caractérisé par le fait qu'**il comporte au moins un détecteur apte à détecter une ouverture d'un capot amovible (82) du générateur (11) et **par le fait que** le dispositif de traitement est configuré pour mémoriser l'ouverture du capot et empêcher qu'en l'absence d'une intervention prédéterminée, effectuée par une personne autorisée, le dispositif de traitement puisse fonctionner à nouveau.

16. Dispositif de traitement selon l'une quelconque des revendications précédentes, **caractérisé par le fait qu'**il comporte un lecteur (17) d'un support d'unités de crédit, par exemple un lecteur de carte à puce.

## Patentansprüche

1. Vorrichtung (10) zur Blitzlichtbehandlung, mit einer Blitzlampe (60) und einem Generator (11), an den die Lampe angeschlossen ist, wobei der Generator (11) mehrere elektronische Schalter (22) aufweist, von denen jeder mit einem zugehörigen Kondensator (21) verbunden ist, wobei jeder elektronische Schalter (22) in einen geöffneten Zustand und in einen geschlossenen Zustand geschaltet werden kann, in dem er es der in dem zugehörigen Kondensator gespeicherten Energie erlaubt, in der Lampe entladen zu werden, **dadurch gekennzeichnet, daß** die Kapazität jedes Kondensators (21) kleiner oder gleich 15 mF ist, damit im Fall einer Störung des betreffenden elektronischen Schalters und der Entladung der Gesamtheit der in dem Kondensator (21) gespeicherten elektrischen Energie der so erzeugte Lichtblitz eine Energiedichte hat, die kleiner oder gleich 30 J/cm² ist, damit keine Gefahr einer unerwünschten Schädigung der behandelten Zone hervorgerufen wird.

2. Behandlungsvorrichtung nach dem vorstehenden Anspruch, **dadurch gekennzeichnet, daß** er eine Steuereinrichtung (30) zur Steuerung der elektronischen Schalter (22) aufweist.

3. Behandlungsvorrichtung nach Anspruch 2, **dadurch gekennzeichnet, daß** die elektronischen Schalter (22) simultan zwischen dem geöffneten und dem geschlossen Zustand umgeschaltet werden.

4. Behandlungsvorrichtung nach einem der Ansprüche 2 und 3, **dadurch gekennzeichnet, daß** die Steuereinrichtung (30) dazu ausgebildet ist, die elektronischen Schalter aus dem geschlossenen Zustand in den geöffneten Zustand umzuschalten, wenn in der Lampe eine vorgegebene Energie entladen worden ist.

5. Behandlungsvorrichtung nach einem der Ansprüche 2 bis 4, **dadurch gekennzeichnet, daß** die Steuereinrichtung so programmierbar ist, daß sie es einem Benutzer erlaubt, die Energie der Blitzlampe zu programmieren, wobei diese Programmierung bewirkt werden kann, indem Tasten (18) gedrückt werden, die jeweils einer Behandlungsart entsprechen.

6. Behandlungsvorrichtung nach einem der vorstehenden Ansprüche, **dadurch gekennzeichnet, daß** sie eine Einrichtung (31) zur Detektion von Isolierungsfehlern aufweist.

7. Behandlungsvorrichtung nach dem vorstehenden Anspruch, **dadurch gekennzeichnet, daß** die Einrichtung (31) zur Detektion von Isolierungsfehlern so konfiguriert ist, daß sie eine Änderung des Potentials an Klemmen von zwei in Serie geschalteten Widerständen (25, 26) detektiert, die einen geerdeten Mittelpunkt sowie Klemmen haben, die mit den beiden Polaritäten einer Spannungsversorgung (20) der Kondensatoren verbunden sind.

8. Behandlungsvorrichtung nach einem der Ansprüche 6 und 7, **dadurch gekennzeichnet, daß** die Einrichtung (31) zur Detektion von Isolierungsfehlern so konfiguriert ist, daß sie einen Leckstrom detektieren kann, der kleiner ist als 100 µA, vorzugsweise kleiner als 50 µA.

9. Behandlungsvorrichtung nach einem der vorstehenden Ansprüche, **dadurch gekennzeichnet, daß** eine elektrisch isolierende Kühlflüssigkeit, insbesondere entmineralisiertes Wasser, dazu benutzt wird, die Lampe zu kühlen, wobei diese Flüssigkeit mit der Erde und einer der beiden Polaritäten der Kondensatoren in elektrischem Kontakt steht.

10. Behandlungsvorrichtung nach einem der vorstehenden Ansprüche, **dadurch gekennzeichnet, daß** sie keine Simmerspanungsversorgung für die Blitzlampe (60) aufweist.

11. Behandlungsvorrichtung nach einem der vorstehenden Ansprüche, **dadurch gekennzeichnet, daß** wenigstens eine sättigungsfähige Spule (50) elektrisch mit der Lampe (60) in Reihe geschaltet ist.

12. Behandlungsvorrichtung nach einem der vorstehenden Ansprüche, **dadurch gekennzeichnet, daß** die Steuereinrichtung dazu ausgebildet ist, eine Information von mindestens einem Fühler (70) aufzunehmen, der in der Lage ist, eine Information zu liefern, die sich auf eine Berührung oder eine Annäherung des Handteils mit der zu behandelnden Zone bezieht und vorzugsweise auch auf den Druck, mit dem das Handteil (12), das die Lampe enthält, gegen die zu behandelnde Zone gedrückt wird.

13. Behandlungsvorrichtung nach einem der vorstehenden Ansprüche, **dadurch gekennzeichnet, daß** sie dazu ausgebildet ist, keine Emission eines Blitzes zuzulassen, solange der von dem Handteil (12) auf die zu behandelnde Zone ausgeübte Druck kleiner oder gleich einem vorbestimmten Grenzwert ist.

14. Behandlungsvorrichtung nach einem der vorstehenden Ansprüche, **dadurch gekennzeichnet, daß** sie dazu ausgebildet ist, die Emission eines Lichtblitzes nicht zuzulassen, solange der Anpreßdruck des Handteils (12) auf die zu behandelnde Zone nicht zwischen vorbestimmten oberen und unteren Grenzwerten für eine gegebene Behandlungsart liegt.

15. Behandlungsvorrichtung nach einem der vorstehenden Ansprüche, **dadurch gekennzeichnet, daß** sie wenigstens einen Detektor aufweist, der dazu ausgebildet ist, das Öffnen einer abnehmbaren Haube (82) des Generators (11) zu detektieren, und daß die Behandlungsvorrichtung dazu konfiguriert ist, das Öffnen der Haube zu speichern und zu verhindern, daß die Behandlungsvorrichtung ohne eine vorbestimmte Intervention, die von einer dazu autorisierten Person vorgenommen wird, wieder in Funktion gesetzt werden kann.

16. Behandlungsvorrichtung nach einem der vorstehenden Ansprüche, **dadurch gekennzeichnet, daß** sie ein Lesegerät (17) für einen Träger für Krediteinheiten, beispielsweise einen Chipkartenleser aufweist.

## Claims

1. Treatment device (10) for the emission of light flashes, comprising a flash lamp (60) and a generator (11) to which the lamp is linked, the generator (11) comprising several electronic switches (22) each associated with a respective capacitor (21), each electronic switch (22) being able to be controlled from a non-conductive state to a conductive state allowing the electrical energy accumulated in the associated capacitor to be discharged in the lamp, **characterised in that** the capacitance of each capacitor (21) is less than or equal to 15 mF so that, in the case of failure of the corresponding electronic switch and discharge in the lamp (60) of all the electrical energy accumulated in the capacitor (21), the light flash thus generated has an energy density of less than or equal to 30 J/cm² so as not to cause a risk of undesirable damage in the treated zone.

2. Treatment device according to the preceding claim, **characterised in that** it comprises a control device (30) to control the electronic switches.

3. Treatment device according to claim 2, **characterised in that** the electronic switches (22) are controlled simultaneously to switch from the non-conductive state to the conductive state.

4. Treatment device according to one of claims 2 and 3, **characterised in that** the control device (30) is configured to switch the electronic switches from the conductive state to the non-conductive state when a predetermined energy has been discharged in the lamp.

5. Treatment device according to any one of claims 2 and 4, **characterised in that** the control device can be programmed so as to allow a user to program the energy of the light flash, where this programming can be performed by pressing keys (18) each corresponding to a type of treatment.

6. Treatment device according to any of the preceding claims, **characterised in that** it comprises an isolation fault detection device (31).

7. Treatment device according to the preceding claim, **characterised in that** the isolation fault detection device (31) is configured to detect a variation in potential at the terminals of two resistors (25, 26) connected in series, with a centre point linked to earth and terminals linked to two poles of a supply (20) of the capacitors.

8. Treatment device according to one of claims 6 and 7, **characterised in that** the isolation fault detection device (31) is configured to detect a leakage current of less than 100 µA, preferably less than 50 µA.

9. Treatment device according to any of the preceding claims, **characterised in that** an electrically isolating cooling liquid, in particular demineralised water, is used to cool the lamp, this liquid coming into electrical contact between earth and one of the two poles of the capacitors.

10. Treatment device according to any of the preceding claims, **characterised in that** there is no electrical supply in simmer mode of the flash lamp (60).

11. Treatment device according to any of the preceding claims, **characterised in that** at least one saturable coil (50) is electrically connected in series with the lamp (60).

12. Treatment device according to any of the preceding claims, **characterised in that** the control device is configured to receive information from at least one sensor (70) able to provide information representative of a contact or proximity of the hand-piece with the zone to be treated, and preferably also representative of the pressure with which a hand-piece (12) containing the lamp is applied to the zone to be treated.

13. Treatment device according to any of the preceding claims, **characterised in that** it is configured to not allow the emission of a flash while the pressure exerted by the hand-piece (12) on the zone to be treated is less than or equal to a predetermined limit.

14. Treatment device according to any of the preceding claims, **characterised in that** it is configured to not allow the emission of a light flash while the application pressure of the hand-piece (12) on the zone to be treated is not between predetermined lower and upper limits for a given type of treatment.

15. Treatment device according to any of the preceding claims, **characterised in that** it comprises at least one detector able to detect an opening of a removable cover (82) of the generator (11) and **in that** the treatment device is configured to memorise the opening of the cover and prevent, in the absence of a predetermined intervention performed by an authorised person, the treatment device from functioning again.

16. Treatment device according to any of the preceding claims, **characterised in that** it comprises a credit unit support reader (17), for example a chipcard reader.
